(19) Europäisches Patentamt
European Patent Office
Office européen des brevets

(11) Veröffentlichungsnummer : **0 290 392 B1**

# (12) EUROPÄISCHE PATENTSCHRIFT

(45) Veröffentlichungstag der Patentschrift :
**17.07.91 Patentblatt 91/29**

(51) Int. Cl.[5] : **C07C 275/54,** A01N 47/34, C07C 217/76, C07C 205/31

(21) Anmeldenummer : **88810276.1**

(22) Anmeldetag : **29.04.88**

## (54) N-Benzoyl-N'-trihalogen-halogenalkoxy-phenylharnstoffe, ihre Herstellung und Verwendung bei der Kontrolle von Schädlingen.

(30) Priorität : **08.05.87 CH 1753/87**

(43) Veröffentlichungstag der Anmeldung :
**09.11.88 Patentblatt 88/45**

(45) Bekanntmachung des Hinweises auf die Patenterteilung :
**17.07.91 Patentblatt 91/29**

(84) Benannte Vertragsstaaten :
**AT BE CH DE ES FR GB GR IT LI NL**

(56) Entgegenhaltungen :
**EP-A- 0 071 279**
**EP-A- 0 194 688**
**EP-A- 0 203 618**
**EP-A- 0 231 152**
**EP-A- 0 235 089**
**EP-A- 0 243 790**
**DE-A- 3 607 298**

(73) Patentinhaber : **CIBA-GEIGY AG**
**Klybeckstrasse 141**
**CH-4002 Basel (CH)**

(72) Erfinder : **Drabek, Jozef, Dr.**
**Benkenstrasse 12**
**CH-4104 Oberwil (CH)**

**EP 0 290 392 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue substituierte N-Benzoyl-N'-2,3,5-trihalogen-4-halogenalkoxy-phenylharnstoffe, ein Verfahren und Zwischenprodukte zu ihrer Herstellung, Arthropodenbekämpfungsmittel, die diese Verbindungen enthalten, sowie ihre Verwendung in der Arthropoden bekämpfung.

Die erfindungsgemässen Verbindungen entsprechen der Formel I

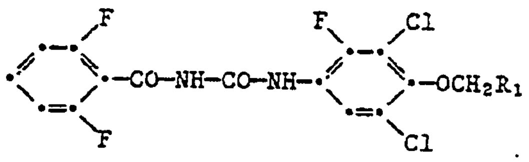

(I),

worin $R_1$ für $CF_3$, $CF_2CF_3$ oder $CF_2CF_2CF_3$ steht.

Unter den Verbindungen der Formel I steht diejenige Verbindung im Vordergrund, in welcher $R_1$ für $CF_2CF_3$ steht.

Die erfindungsgemässen Verbindungen können nach an sich bekannten Verfahren hergestellt werden. Solche Verfahren sind u.a. in den DE-OS 21 23 236, 26 01 780 und 32 40 975 beschrieben. So können die Verbindungen der Formel I z.B. erhalten werden, indem man

a) ein Anilin der Formel II

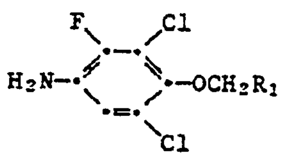

(II)

mit dem Benzoylisocyanat der Formel III

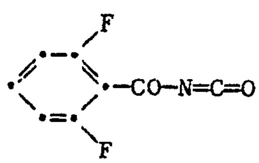

(III),

oder

b) ein Isocyanat der Formel IV

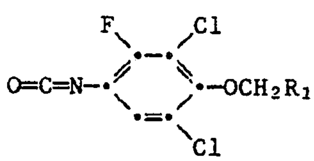

(IV)

mit dem Benzamid der Formel V

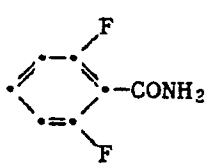

(V),

oder

c) ein Anilin der Formel II mit einem Urethan der Formel VI

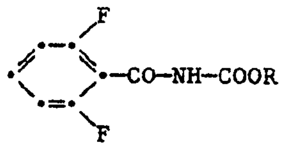

(VI)

umsetzt. In den Formeln II und IV hat $R_1$ die für Formel I angegebene Bedeutung und in Formel VI steht R für einen $C_1$-$C_8$-Alkylrest, der gegebenenfalls mit Halogen, vorzugsweise Chlor, substituiert sein kann.

Die erwähnten Verfahren a), b) und c) können vorzugsweise unter normalem Druck und in Gegenwart eines organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran ; N,N-dialkylierte Carbonsäureamide ; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol ; Nitrile, wie Acetonitril oder Propionitril ; Dimethylsulfoxid sowie Ketone, z.B. Aceton, Metyläthylketon, Methylisopropylketon und Methylisobutylketon. Verfahren a) wird im allgemeinen bei einer Temperatur von — 10 bis +200°C, vorzugsweise zwischen 0 und 100°C, z.B. bei Raumtemperatur, gegebenenfalls in Gegenwart einer organischen Base, z.B. Triäthylamin, durchgeführt. Die Durchführung von Verfahren b) erfolgt bei einer Temperatur von 0 bis 150°C, vorzugsweise beim Siedepunkt des verwendeten Lösungsmittels, und gegebenenfalls in Gegenwart einer organischen Base, wie Pyridin, und/oder unter Zusatz eines Alkali-oder Erdalkalimetalls, vorzugsweise Natrium. Für das Verfahren c), d.h. für die Umsetzung der Urethane der Formel VI mit einem Anilin der Formel II, werden Temperaturen zwischen etwa 60°C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln III bis VI sind bekannt oder können analog bekannten Verfahren hergestellt werden.

Bei den Ausgangsstoffen der Formel II handelt es sich um neuartige Verbindungen, die ebenfalls einen Gegenstand der vorliegenden Erfindung bilden. Die Verbindungen der Formel II können in an sich bekannter Weise z.B. dadurch hergestellt werden, dass man ein entsprechend substituiertes Nitrobenzol der Formel VII

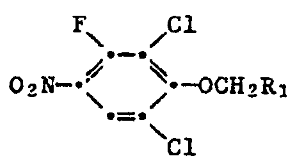

(VII)

in Analogie zu dem in J. Org. Chem. 29 (1964), 1, aufgezeigten Verfahren hydriert (vgl. auch die dort zitierte Literatur). Aber auch durch chemische Reduktion (z.B. mittels Sn-(II)-Chlorid/HCl) der entsprechenden Nitroverbindungen der Formel VII sind Aniline der Formel II zugänglich (Vgl. Houben Weyl, "Methoden d. org. Chemie" 11/1, 422). Eine weitere Herstellungsmöglichkeit für Aniline der Formel II besteht darin, dass man das gegebenenfalls acylierte 2-Fluor-3,5-dichlor-4-hydroxyanilin entsprechend haloalkyliert und dann gegebenenfalls die Acylgruppe entfernt, z.B. durch saure Hydrolyse.

Die Nitroverbindungen der Formel VII sind ebenfalls neu und bilden einen Gegenstand der vorliegenden Erfindung. Sie sind z.B. herstellbar durch entsprechende Haloalkylierung des 2-Fluor-3,5-dichlor-4-Nitrophenols (vgl. französische Patentschrift 2,005,876) oder durch Umsetzen von 2,4-Difluor-3,5-dichlor-nitrobenzol mit einem entsprechenden Polyfluoralkanol in alkalischer Lösung und Dimethylsulfoxid als Lösungsmittel (vgl. "The Chemistry of the Hydroxyl Group", Seiten 83-124 ; Interscience Publishers Inc., New York, 1971).

Zum Benzoylisocyanat der Formel III kann man unter anderem wie folgt gelangen (vgl. J. Agr. Food Chem. 21, 348 und 993 ; 1973) :

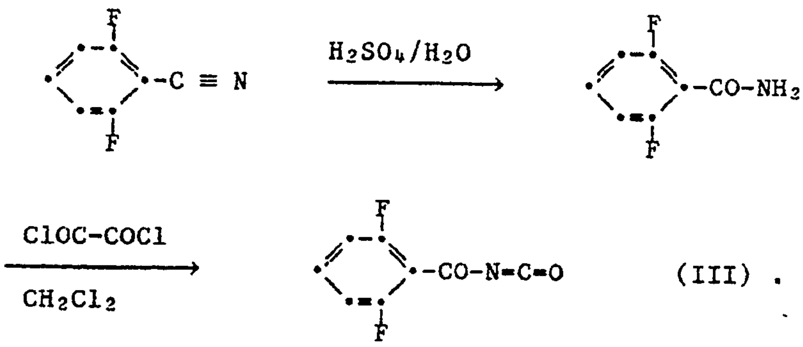


(III) .

Die 4-(Polyfluoralkoxy)-phenylisocyanate der Formel IV lassen sich z.B. durch Phosgenierung eines Anilins der Formel II nach allgemein üblichen Verfahren herstellen. Das ebenfalls als Ausgangsstoff zu verwendende Benzamid der Formel V ist bekannt (vgl. z.B. Beilstein "Handbuch der organischen Chemie" Bd. 9, S. 336).

Das Urethan der Formel VI kann in an sich bekannter Weise erhalten werden durch Umsetzung das Benzoylisocyanats der Formel III mit einem entsprechenden Alkohol oder durch Umsetzung des Benzamides der Formel V in Anwesenheit einer basischen Verbindung mit einem entsprechenden Ester der Chlorameisensäure Cl-COOR.

In der veröffentlichten europäischen Patentanmeldung 0 071 279 werden ganz allgemein u.a. N-Halogenbenzoyl-N'-(halogen-4-halogenalkoxy)-phenylharnstoffe als Insektizide mit larvizider Wirkung beschrieben. In der veröffentlichten europäischen Patentanmeldung 0 194 688 werden ebenfalls ganz allgemein N-Dihalogenbenzoyl-N'-(halogen-4-fluoralkoxy)-phenylharnstoffe als Insektizide mit ovizider und larvizider Wirkung beschrieben. In keiner der beiden Veröffentlichungen werden jedoch N-(2,6-Difluorbenzoyl,)-N'-(2-fluor-3,5-dichlor-4-trifluoräthoxy oder -pentafluorpropoxy oder -heptafluorbutoxy)-phenylharnstoffe spezifisch offenbart.

Ueberraschenderweise wurde gefunden, dass die erfindungsgemässen Verbindungen der Formel I bei günstiger Warmblüter- und Pflanzenverträglichkeit stärkere Wirkstoff in der Arthropodenbekämpfung sind. So eignen sich die Verbindungen der Formel I z.B. zur Bekämpfung von Arthropoden an Tieren und Pflanzen. Solche Schädlinge gehören insbesondere zu den Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und zu den Arachniden der Ordnung Acarina, wie z.B. Milben und Zecken. Dabei kann jedes Entwicklungsstadium der Arthropoden bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formel I von Imagines aufgenommen, so kann sich ihre Wirkung in unmittelbarer Abtötung der Arthropoden zeigen oder aber in verminderter Eiblage und/oder Schlupfrate. Die letztere Erscheinung kann insbesondere bei Coleopteren beobachtet werden. Bei der Bekämpfung von tierparasitären Arthropoden, insbesondere an Haus- und Nutztieren, kommen vor allem Ektoparasiten, wie z.B. Milben und Zecken und Dipteren, wie z.B. Lucilia sericata in Betracht.

Insbesondere zeichnen sich die erfindungsgemässen Verbindungen durch eine ausgezeichnete larvizide Wirkung bei Spodoptera littoralis und Heliothis virescens aus.

Die gute pestizide Wirkung der erfindungsgemässen Verbindungen der Formel I entspricht einer Abtötungsrate (Mortalität) von mindestens 50-60% der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen, inerten, pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die einen Wirkstoff der Formel I, bzw. Kombinationen dieser Wirkstoffe mit anderen Insektiziden oder Akariziden, und gegebenenfalls einen festen oder flüssigen Zusatzstoff enthalenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbezolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome in Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979 ;

Dr. Helmut Stache "Tensid Taschenbuch", Carl Hanser Verlag München/Wien 1981.

Die pestiziden Zubereitungen enthalten in der Regel 0,1 bis 99%, insbesondere 0,1 bis 95%, Wirkstoff der Formel I oder Kombinationen dieser Wirkstoffe mit andern Insektiziden oder Akariziden, 1 bis 99,9% eines festen oder flüssigen Zusatzstoffes und 0 bis 25%, insbesondere 0,1 bis 20%, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Beispiel 1 : Herstellung

### 1.1. Zwischenprodukte

### 1.1.1. Nitrobenzole

#### 1.1.1.1. 2-Fluor-3,5-dichlor-4-(2',2',3',3',3'-pentafluorpropoxy)-nitrobenzol

12,4 g 90%iges Kaliumhydroxid werden in 80 ml Dimethylsulfoxid suspendiert. Zu dieser Suspension werden tropfenweise unter Rühren 30 g 2',2',3',3',3'-Pentafluorpropanol gegeben. Die entstandene Lösung lässt man unter Rühren bei Raumtemperatur in eine Lösung von 52,4 g 2,4-Difluor-3,5-dichlor-nitrobenzol in 150 ml Dimethylsulfoxid tropfen. Nach beendeter Zugabe wird noch 2 Stunden bei Raumtemperatur weitergerührt. Alsdann wird das Reaktionsgemisch eingeengt, das Rohprodukt in Methylenchlorid gelöst, die Lösung mit Wasser gewaschen und getrocknet und schliesslich das Lösungsmittel abdestilliert. Die Reinigung erfolgt chromatographisch an einer Kieselgelsäule mit Hexan/Aether 19 : 1 als Laufmittel. Nach Abdestillation des Lösungsmittels liegt die Titelverbindung der Formel

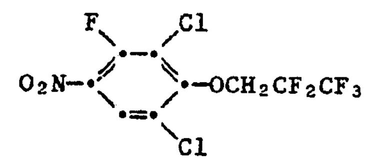 (Verb. 1.1.1.1.)

in Form gelber Kristalle vor ; Smp. 62-63°C.

Auf analoge Weise werden die folgenden Verbindungen hergestellt :

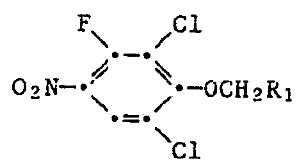

| Verb. Nr. | $R_1$ | Smp. °C |
|---|---|---|
| 1.1.1.2 | $CF_3$ | 50 - 51 |
| 1.1.1.3 | $CF_2CF_2CF_3$ | 54 - 55 |

### 1.1.2. Aniline

#### 1.1.2.1. 2-Fluor-3,5-dichlor-4-(2',2',3',3',3'-pentafluorpropoxy)-anilin

6,2 g des obigen Nitrobenzols werden in 60 ml Tetrahydrofuran gelöst und bei Raumtemperatur während 8 Stunden in Gegenwart von 2 g Raney-Nickel hydriert ($H_2$-Aufnahme ; 1,2 l). Das Reaktionsgemisch wird filtriert, das Lösungsmittel abdestilliert und der Rückstand destilliert. Die Titelverbindung der Formel

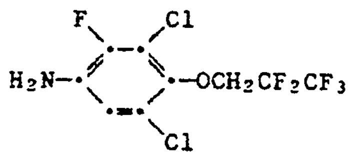

(Verb. Nr. 1.1.2.1.)

weist einen Siedepunkt von 170°C/0,06 Torr auf.

Auf analoge Weise werden die folgenden Verbindungen hergestellt:

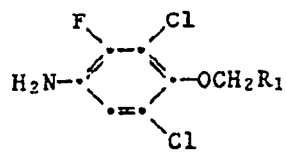

| Verb. Nr. | $R_1$ | phys. Daten |
|---|---|---|
| 1.1.2.2 | $CF_3$ | Smp. 32-35°C |
| 1.1.2.3 | $CF_2CF_2CF_3$ | Sdp. 140°C/0,03 Torr |

## 1.2. Endprodukte

### 1.2.1. N-(2,6-Difluorbenzoyl)-N'-[2-fluor-3,5-dichlor-4-(2',2',3',3',3'-pentafluorpropoxy)-phenyl]-harnstoff

6 g 2-Fluor-3,5-dichlor-4-(2',2',3',3',3'-pentafluorpropoxy)-anilin, gelöst in 50 ml trockenem Toluol, werden bei Raumtemperatur mit 3,3 g 2,6-Difluorbenzoylisocyanat, gelöst in 10 ml trockenem Toluol, versetzt und 10 Stunden lang gerührt. Anschliessend werden etwa 75% des Lösungsmittels am Rotationsverdampfer entfernt. Der entstandene Niederschlag wird abgenutscht, mit wenig kaltem Toluol und Hexan gewaschen und im Vakuum getrocknet. Die Titelverbindung der Formel

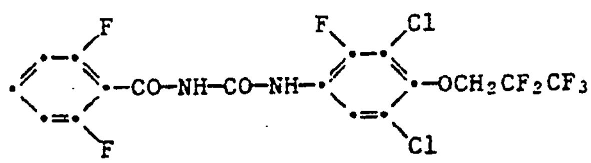

(Verb. Nr. 1.2.1.)

liegt als weisses, kristallines Pulver vor; Smp. 176-179°C.

Auf analoge Weise werden die folgenden Verbindungen hergestellt:

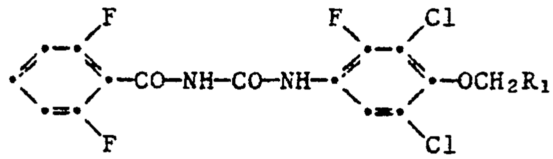

| Verb. Nr. | $R_1$ | Smp. °C |
|---|---|---|
| 1.2.2 | $CF_3$ | 192-193 |
| 1.2.3 | $CF_2CF_2CF_3$ | 163-164 |

Beispiel 2 : Formulierungen von Wirkstoffen der Formel I gemäss Herstellungsbeispiel 1.2

(% = Gewichtsprozent)

| 2.1 Emulsions-Konzentrat | |
|---|---|
| Wirkstoff gemäss dem Herstellungsbeispiel 1.2 | 10 % |
| Ricinusöl-polyäthylenglykoläther (36 Mol AeO) | 25 % |
| Butanol | 15 % |
| Essigester | 50 % |

Aus diesem Konzentrat können durch Verdünnen mit Wasser Emulsionen jeder gewünschten Konzentration hergestellt werden.

| 2.2 Lösungen | a) | b) |
|---|---|---|
| Wirkstoff gemäss dem Herstellungsbeispiel 1.2 | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190°C) | - | 74 % |

Die Lösungen sind zur Anwendung in Form kleinster Tropfen geeignet.

| 2.3 Granulate | a) | b) |
|---|---|---|
| Wirkstoff gemäss dem Herstellungsbeispiel 1.2 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Extruder Granulat | |
|---|---|
| Wirkstoff gemäss dem Herstellungsbeispiel 1.2 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.5 Umhüllungs-Granulat | |
|---|---|
| Wirkstoff gemäss dem Herstellungsbeispiel 1.2 | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig anfgetragen. Auf diese Weise erhält man staubfreie Umhüllungs-Granulate.

| 2.6 Stäubemittel | a) | b) | c) | d) |
|---|---|---|---|---|
| Wirkstoff gemäss dem Herstellungsbeispiel 1.2 | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

| 2.7 Spritzpulver | a) | b) | c) |
|---|---|---|---|
| Wirkstoff gemäss dem Herstellungsbeispiel 1.2 | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

2.8 Suspensions-Konzentrat

| | |
|---|---|
| Wirkstoff gemäss dem Herstellungsbeispiel 1.2 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3 : Biologische Prüfungen

3.1 Wirkung gegen Musca domestica

Je 50 g frisch zubereitetes CSMA-Nährsubstrat für Maden werden in einen Becher eingewogen. Von einer 1 gew.%igen acetonischen Lösung des Wirkstoffes werden 5 ml auf das im Becher befindliche Nährsubstrat pipettiert. Nach dem Durchmischen des Substrates lässt man das Aceton mindestens 20 Stunden lang verdampfen.

Dann werden 25 eintägige Maden von Musca domestica in den das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in einem mit einem Siebdeckel verschlossenen Gefäss deponiert.

Die ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen gemäss Beispiel 1.2 zeigen gute Wirkung im obigen Test.

3.2 Wirkung gegen Lucilia sericata

Zu 9 ml eines Zuchtmediums wird bei 50°C 1 ml einer 0,5% Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss Beispiel 1.2 zeigen in diesem Test gute Wirkung gegen Lucilia sericata.

3.3 Wirkung gegen Aedes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 12,5 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss Beispiel 1.2 zeigen gute Wirkung in diesem Test.

3.4 Frassgift-Wirkung auf Spodoptera littoralis- und Heliothis virescens-Larven ($L_1$)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsionen 3, 1,5 0,8, 0,4 und 0,2 ppm der zu prüfenden Verbindung enthält.

Nach dem Antrocknen des Belages wird jede Baumwollpflanze mit Spodoptera littoralis- und Heliothis virescens-Larven im ersten larvalen Stadium besetzt. Der Versuch wird bei 26°C und ca. 50% relativer Luftfeuchtigkeit durchgeführt. Nach 6 Tagen wird die Mortalität der angesetzten Larven bestimmt.

| | | | Mortalität in % nach 6 Tagen und bei | | | | |
|---|---|---|---|---|---|---|---|
| | | | 3 ppm | 1,5 ppm | 0,8 ppm | 0,4 ppm | 0,2 ppm |
| A | 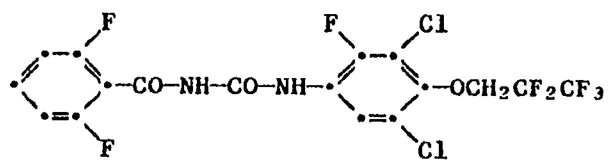 Verb. Nr. 1.2.1. | Spodoptera littoralis $L_1$ | 100 | 100 | 100 | 100 | 80 |
| | | Heliothis virescens $L_1$ | 100 | 100 | 90 | 53 | 0 |
| B | 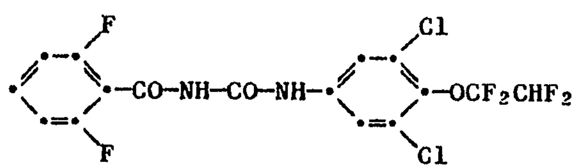 Europäische Patentanmeldung 0 071 279 Verb. Nr. 48 | Spodoptera littoralis $L_1$ | 100 | 97 | 70 | 0 | 0 |
| | | Heliothis virescens $L_1$ | 100 | 67 | 7 | 0 | 0 |

Die Verbindung Nr. 1.2.1. gemäss Beispiel 1.2. zeigt bei gleicher Konzentration eine bedeutend stärkere Wirkung.

3.5 Frassgift-Wirkung auf Spodoptera littoralis- und Heliothis virescens-Larven ($L_3$)

Eingetopfte Sojapflanzen (Topfgrösse 10 cm Durchmesser) im 4-Blatt-Stadium werden mit wässrigen Wirkstoffemulsionen besprüht, die den Wirkstoff in einer Konzentration von 0,75 ppm enthalten.

Nach zwei Tagen werden die behandelten Sojapflanzen mit je 10 Larven von Spodoptera littoralis und Heliothis virescens im dritten larvalen Stadium besiedelt. Der Versuch wird bei 26°C und ca. 60% relativer Luftfeuchtigkeit im Dämmerlicht durchgeführt. Die Bonitur erfolgt nach 2 und 5 Tagen ; es wird die Mortalität der Larven in Prozenten bestimmt.

Verbindungen gemäss Beispiel 1.2. zeigen bei Spodoptera und bei Heliothis nach 2 und 5 Tagen 80-100%ige Wirkung (Mortalität).

3.6 Frassgift-Wirkung auf Plutella xylostella-Larven ($L_2$)

Eingetopfte Chinakohlpflanzen (Topfgrösse 10 cm Durchmesser) im 4-Blatt-Stadium werden mit einer wässrigen Wirkstoffemulsion besprüht, die den Wirkstoff in einer Konzentration von 0,75 ppm enthält.

Nach zwei Tagen werden die behandelten Chinakohlpflanzen mit 10 Plutella xylostella-Larven im zweiten larvalen Stadium besiedelt. Der Versuch wird bei 26°C und ca. 60% relativer Luftfeuchtigkeit im Dämmerlicht durchgeführt. Die Bonitur erfolgt nach 2 und 5 Tagen ; es wird die Mortalität der Larven in Prozenten bestimmt.

Verbindungen gemäss Beispiel 1.2. zeigen nach 2 und 5 Tagen 80-100%ige Wirkung (Mortalität).

3.7 Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 400 ppm des zu prüfenden Wirkstoffes, eingetaucht. Nach dem Eintrocknen der Lösung wird das Filterpapier mit den Eiern in einer Petrischale ausgelegt und bei einer Temperatur von 28°C belassen. Nach 6 Tagen wird der prozentuale Schlupf aus den behandelten Eiern bewertet.

Verbindungen gemäss Beispiel 1.2 zeigen gute Wirkung in obigem Test.

3.8 Reproduktions-Beeinflussung von Anthonomus grandis

Adulte Anthonomus grandis, die nach dem Schlupf nicht älter als 24 Stunden sind, werden in Gruppen von jeweils 25 Käfern in Käfige mit Gitterwänden überführt. Die mit den Käfern besetzten Käfige werden sodann während 5 bis 10 Sekunden in eine acetonische Lösung, enthaltend 0,1 Gew.% des zu prüfenden Wirkstoffes, eingetaucht.

Wenn die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraumes von etwa vier Wochen überprüft. Die Bonitierung erfolgt anhand der Verminderung der Anzahl abgelegter Eier und der daraus geschlüpften Larven im Vergleich zu unbehandelten Kontrollen.

Verbindungen gemäss Beispiel 1.2 zeigen eine gute reproduktionsreduzierende Wirkung im obigen Test.

3.9 Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60% relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie

der Antifeedant-Wirkung gegenüber unbehandelten Kontrollansätzen.

Verbindungen gemäss Beispiel 1.2 zeigen gute Wirkung in diesem Test.

3.10 Wirkung gegen Epilachna varivestis

Etwa 15-20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28°C und 60% relativer Luftfeuchtigkeit durchgeführt.

Nach 2 und 3 Tagen wird die Mortalität in Prozenten bestimmt. Zur Auswertung hinsichtlich allfälligem Frassschaden (Antifeeding-Effekt), Entwicklungs- und Häutungsstörungen werden die Versuchstiere während weiterer 3 Tage beobachtet.

Verbindungen gemäss Beispiel 1.2 zeigen gute Wirkung im obigen Test.

3.11 Ovizide Wirkung auf Heliothis virescens und Spodoptera littoralis

Entsprechende Mengenanteile einer benetzbaren pulverförmigen Formulierung, enthaltend 25 Gew.% des zu prüfenden Wirkstoffes, werden mit jeweils soviel Wasser vermischt, dass sich eine wässrige Emulsion mit einer Wirkstoffkonzentration von 400 ppm ergibt.

In diese wirkstoffhaltige Emulsion werden eintägige auf Cellophan abgelegte Eigelege von Heliothis und auf Papier abgelegte Eigelege von Spodoptera während drei Minuten eingetaucht und dann auf Rundfilter abgenutscht. Die so behandelten Gelege werden in Petrischalen ausgelegt und bei 28°C und 60% relativer Luftfeuchtigkeit in der Dunkelheit aufbewahrt. Nach 5 bis 8 Tagen wird die Schlupfrate, d.h. die Anzahl Larven, die sich aus den behandelten Eiern entwickelt haben, gegenüber unbehandelten Kontrollansätzen bestimmt.

Verbindungen gemäss Beispiel 1.2 zeigen in obigem Test eine 80-100%ige ovizide Wirkung (Mortalität) gegen Heliothis virescens und Spodoptera littoralis.

## Patentansprüche

1. Verbindungen der Formel I

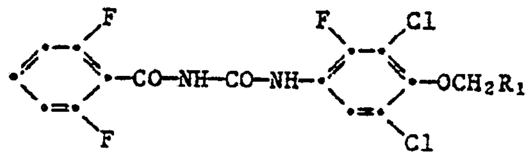

(I),

worin $R_1$ für $CF_3$, $CF_2CF_3$ oder $CF_2CF_2CF_3$ steht.

2. Die Verbindungen der Formel

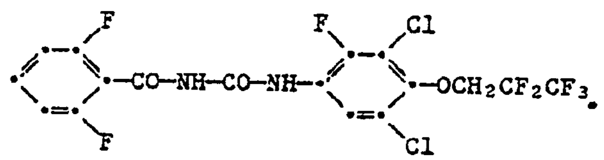

3. Verbindungen der Formel II

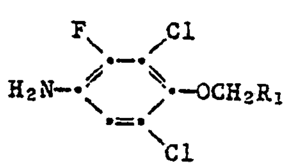

(II),

worin $R_1$ für $CF_3$, $CF_2CF_3$ oder $CF_2CF_2CF_3$ steht.

4. Verbindungen der Formel VII

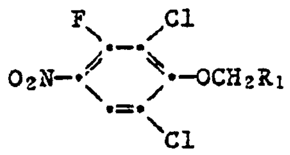

(VII),

worin $R_1$ für $CF_3$, $CF_2CF_3$ oder $CF_2CF_2CF_3$ steht.

5. Verfahren zur Herstellung einer Verbindung der Formel I

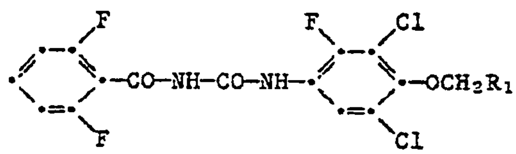

(I),

worin $R_1$ für $CF_3$, $CF_2CF_3$ oder $CF_2CF_2CF_3$ steht, dadurch gekennzeichnet, dass man

a) ein Anilin der Formel II

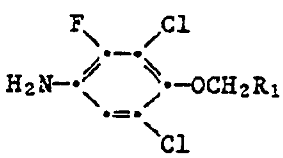

(II)

mit dem Benzoylisocyanat der Formel III

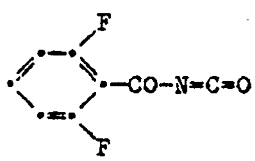

(III), oder

b) ein Isocyanat dar Formel IV

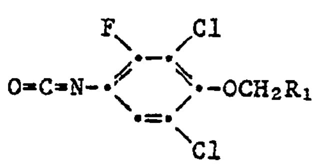

(IV)

mit dem Benzamid der Formel V

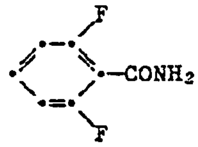

(V), oder

c) ein Anilin der Formel II mit einem Urethan der Formel VI

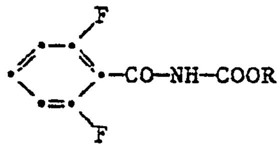

(VI)

umsetzt, wobei in den Formeln II und IV $R_1$ die angegebene Bedeutung hat und in Formel VI R für einen gegebenenfalls halogenierten $C_1$-$C_8$-Alkylrest steht.

6. Arthropodenbekämpfungsmittel, welches als aktive Komponente eine Verbindung der Formel I

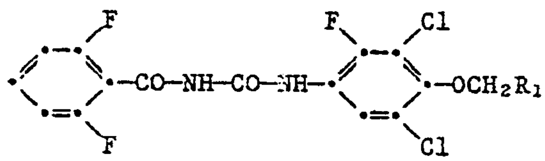

(I),

enthält, worin $R_1$ für $CF_3$, $CF_2CF_3$ oder $CF_2CF_2CF_3$ steht, zusammen mit geeigneten Trägern und/oder Zuschlagstoffen.

7. Verwendung einer Verbindung der Formel I

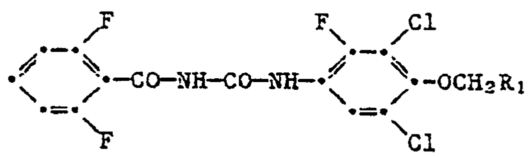

(I),

worin $R_1$ für $CF_3$, $CF_2CF_3$ oder $CF_2CF_2CF_3$ steht, zur Bekämpfung von Tiere und Pflanzen, befallenden Arthopoden auf oder in ihren Lebensränmen mit Augnahme des menschlichen und tierischen Körpers.

8. Verwendung gemäss Anspruch 7 einer Verbindung der Formel I zur Bekämpfung von Insekten und Arachniden.

9. Verwendung gemäss Anspruch 8 einer Verbindung der Formel I zur Bekämpfung larvaler Stadien von pflanzenschädigenden Insekten.

10. Verfahren zum Bekämpfen von Arthropoden an Tieren und Pflanzen auf oder in ihren Lebensränmen mit Ausnahme menschlicher und tierischer Körper, dadurch gekennzeichnet, dass man die Arthopoden in ihren verschiedenen Entwicklungsstadiem mit einer Verbindung der Formel I

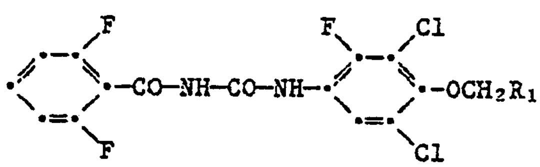

(I),

worin $R_1$ für $CF_3$, $CF_2CF_3$ oder $CF_2CF_2CF_3$ steht, in Kontakt bringt.

**Claims**

1. A compound of formula I

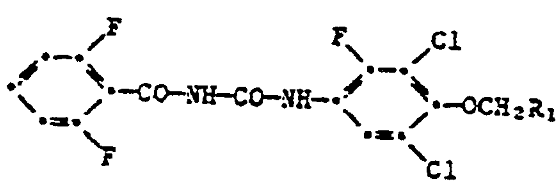

(I)

wherein $R_1$ is $CF_3$, $CF_2CF_3$ or $CF_2CF_2CF_3$.

2. A compound of formula

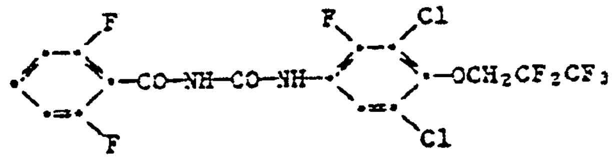

3. A compound of formula II

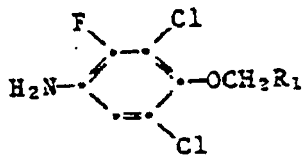

(II)

wherein $R_1$ is $CF_3$, $CF_2CF_3$ or $CF_2CF_2CF_3$.

4. A compound of formula VII

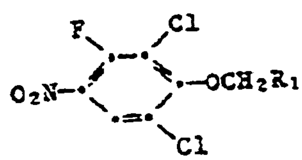

(VII),

wherein $R_1$ is $CF_3$, $CF_2CF_3$ or $CF_2CF_2CF_3$.

5. A process for the preparation of a compound of formula I

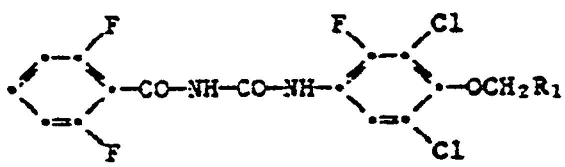

(I),

wherein $R_1$ is $CF_3$, $CF_2CF_3$ or $CF_2CF_2CF_3$, which comprises

a) reacting an aniline of formula II

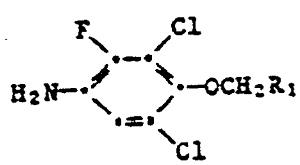

(II)

with the benzoyl isocyanate of formula III

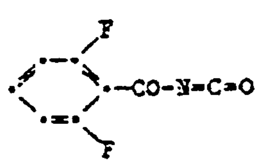

(III),

or

b) reacting an isocyanate of formula IV

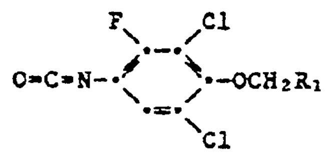

(IV)

with the benzamide of formula V

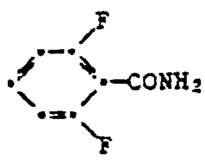

(V)

or

c) reacting an aniline of formula II with a urethane of formula VI

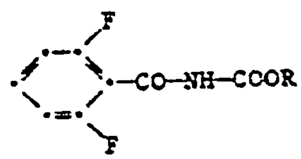

(VI),

wherein in formulae II and IV $R_1$ has the meaning given and, in formula VI, R is a $C_1$-$C_8$alkyl radical which is unsubstituted or substituted by halogen.

6. A composition for the control of arthropods which comprises, as active component, a compound of formula I

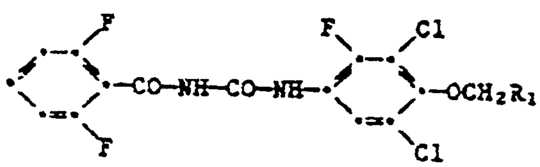

(I),

wherein $R_1$ is $CF_3$, $CF_2CF_3$ or $CF_2CF_2CF_3$, together with suitable carriers and/or adjuvants.

7. The use of a compound of formula I

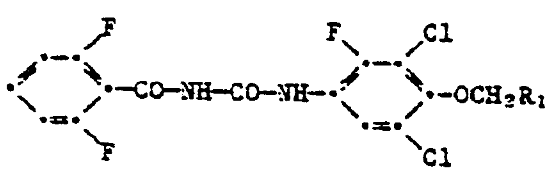

(I),

wherein $R_1$ is $CF_3$, $CF_2CF_3$ or $CF_2CF_2CF_3$, for controlling animal- and plant-attacking arthropods on or in their habitats with the exception of the human or animal body.

8. The use according to claim 7 of a compound of formula I for controlling insects and arachnids.

9. The use according to claim 8 of a compound of formula I for controlling larval stages of plant-destructive insects.

10. A method of controlling arthropods attacking animals and plants on or in their habitats, with the exception of the human or animal body, wherein arthropods in their various stages of development are brought into contact with a compound of formula I

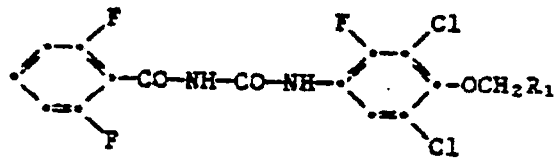
(I),

wherein $R_1$ is $CF_3$, $CF_2CF_3$ or $CF_2CF_2CF_3$.

## Revendications

1. Composés de formule I

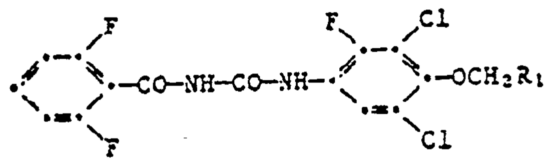
(I),

où $R_1$ représente $CF_3$, $CF_2CF_3$ ou $CF_2CF_2CF_3$.

2. Composé de formule

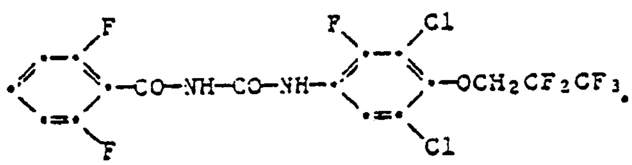

3. Composés de formule II

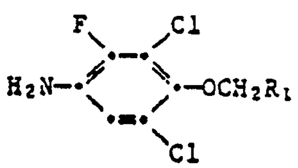
(II),

où $R_1$ représente $CF_3$, $CF_2CF_3$ ou $CF_2CF_2CF_3$.

4. Composés de formule VII

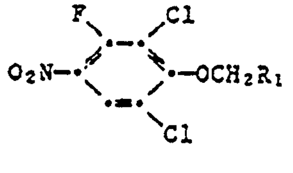
(VII),

où $R_1$ représente $CF_3$, $CF_2CF_3$ ou $CF_2CF_2CF_3$.

5. Procédé de préparation d'un composé de formule I

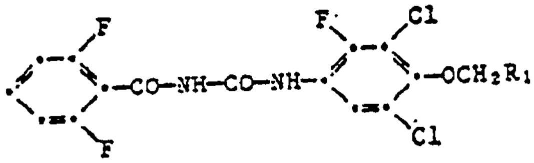
(I),

où $R_1$ représente $CF_3$, $CF_2CF_3$ ou $CF_2CF_2CF_3$, caractérisé en ce que

a) on fait réagir une aniline de formule II

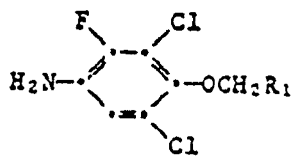

(II)

avec l'isocyanate de benzoyle de formule III

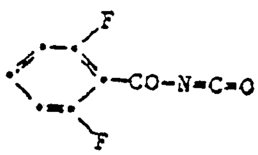

(III),

ou

b) on fait réagir un isocyanate de formule IV

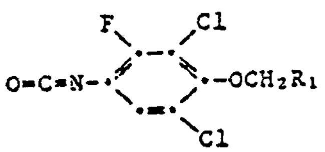

(IV)

avec le benzamide de formule V

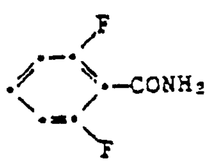

(V),

ou

c) on fait réagir une aniline de formule II avec un uréthane de formule VI

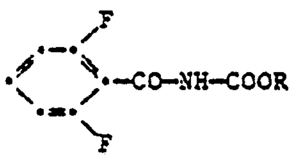

(VI)

où, dans les formules II et IV, $R_1$ a la signification indiquée et dans la formule VI, R représente un radical alcoyle en $C_{1-8}$ éventuellement halogéné.

6. Agent de lutte contre les Arthropodes qui contient comme composant actif un composé de formule I

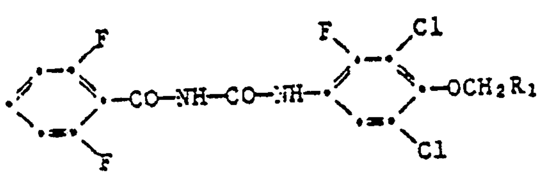

(I),

où $R_1$ représente $CF_3$, $CF_2CF_3$ ou $CF_2CF_2CF_3$, avec des supports et/ou additifs appropriés.

7. Application d'un composé de formule I

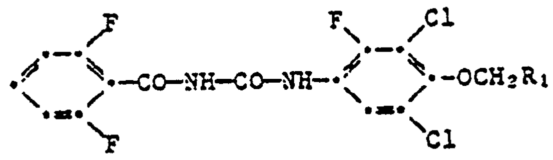

(I),

où $R_1$ représente $CF_3$, $CF_2CF_3$ ou $CF_2CF_2CF_3$, à la lutte contre les Arthropodes attaquant les animaux et les plantes ou à l'attaque de leur espace vital à l'exception du corps humain ou animal.

8. Application selon la revendication 7 d'un composé de formule I à la lutte contre les Insectes et les Arachnides.

9. Application selon la revendication 8, d'un composé de formule I à la lutte contre les stades larvaires d'insectes causant des dommages aux plantes.

10. Application à la lutte contre les Arthropodes chez les animaux et les plantes ou contre leur espace vital à l'exception du corps humain ou animal, caractérisée en ce qu'on met les Arthropodes à leurs divers stades de développement au contact avec un composé de formule I

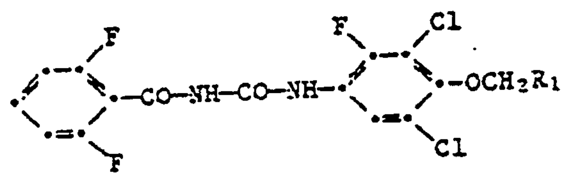

(I),

où $R_1$ représente $CF_3$, $CF_2CF_3$ ou $CF_2CF_2CF_3$.